## Europäisches Patentamt

(19) **European Patent Office**

**Office européen des brevets**

(11) Publication number: **0 085 813**
**B1**

(12) **EUROPEAN PATENT SPECIFICATION**

(45) Date of publication of patent specification: **11.06.86**

(51) Int. Cl.⁴: **A 61 B 5/10, G 01 N 3/42**

(21) Application number: **82306848.1**

(22) Date of filing: **21.12.82**

(54) Tubular probe for sensing hardness variations when rubbed over a surface to be examined.

(30) Priority: **28.12.81 JP 212757/81**
**28.12.81 JP 212759/81**
**28.12.81 JP 212760/81**

(43) Date of publication of application:
**17.08.83 Bulletin 83/33**

(45) Publication of the grant of the patent:
**11.06.86 Bulletin 86/24**

(84) Designated Contracting States:
**AT BE CH DE FR GB IT LI NL SE**

(56) References cited:
**FR-A-2 391 695**
**JP-U-75 001 837**
**US-A-4 132 224**
**US-A-4 159 640**

(73) Proprietor: **OLYMPUS OPTICAL CO., LTD.**
**43-2, 2-chome, Hatagaya Shibuya-ku**
**Tokyo 151 (JP)**

(72) Inventor: **Shishido, Yoshio**
**No. 2-1-30-512, Fuchinobe**
**Sagamihara-shi Kanagawa-ken (JP)**

(74) Representative: **Kennedy, Victor Kenneth et al**
**G.F. REDFERN & CO. Marlborough Lodge 14**
**Farncombe Road**
**Worthing West Sussex BN11 2BT (GB)**

Courier Press, Leamington Spa, England.

## Description

This invention relates to a hardness measuring probe by which variations in the hardness of an object to be examined can be continuously and quickly measured by a simple operation.

Recent medical and veterinary developments have led to the extensive use of endoscopes which enable any irregularity of an affected part or the state of a tumor to be observed via an elongate insertion sheath introduced into a body cavity or to permit a therapeutic treatment to be made, if required by using a forceps.

However a viewing device such as an endoscope does not in itself enable the hardness of a tumor or the state of a node in an effected part to be detected.

The hardness of the tumor in the above mentioned affected part will depend on its type and its growth stage. Therefore, if the degree of hardness can be detected and used as diagnostic data, the resultant diagnosis will be made more accurate.

It has been proposed in the prior art to use a pressure-sensitive element provided at the tip of a flexible bar-shaped member which can be advanced from and withdrawn into a tubular member such as a forceps channel in an endoscope, as is disclosed in the Gazette of Japanese Utility Model Publication No 18371/1975.

In order to detect any variations of hardness in and around the normal part and affected part by measurements of the hardness around an object position using the above mentioned prior art example, the result of successive measurements at a single place will vary, depending on the degree of ambient pressure applied to the surface, and there has been a need to repeat the contacting and separating operations.

The above described prior art examples has a further problem in that when measuring the hardness of an affected part or the like within a body cavity inflated with air, the influences of atmospheric pressure around an affected part may vary during the measurement, and the effect of the atmospheric pressure acting on the pressure-sensitive element itself will appear as a variation in the measured result, and the pressure applied at the affected part will appear to be varied in the measured result.

Objects of the present invention are to provide a probe making it possible to sense the hardness differences in and around an object position by a simple operation, continuously with high precision, yet allowing any influence of the atmospheric pressure around an object to be sensed to be taken into consideration.

According to the present invention, there is provided a tubular probe for sensing hardness variations. When rubbed over a surface to be examined comprising pressure sensor means to detect the respective difference in pressures applied at two pressure receiving surfaces, a slidable shaft being provided at the distal end of the tubular probe to transmit the force applied to its tip by the rubbing to an inner end acting directly or indirectly on a first of said pressure receiving surfaces, arranged to face the inner end of said shaft, and an aperture on the peripheral surface of the probe communicating the ambient pressure around the probe with the second of said pressure receiving surfaces.

The invention will now be described with reference to the drawings in which:—

Figure 1 is a schematic explanatory view showing a method of measuring hardness variations used in a first exemplary embodiment;

Figure 2 is an explanatory view showing a display of data as measured by the first exemplary embodiment;

Figure 3 is a vertical section showing structural details of the first exemplary embodiment;

Figure 4 is a plan view of the first embodiment;

Figure 5 is an explanatory view showing the basic structure of the pressure sensor used in the first embodiment;

Figure 6 is a vertical section showing details of the tip of a second exemplary embodiment of the present invention; and

Figure 7 is a vertical section showing details of the tip of a piston shaft in a third exemplary embodiment of the present invention.

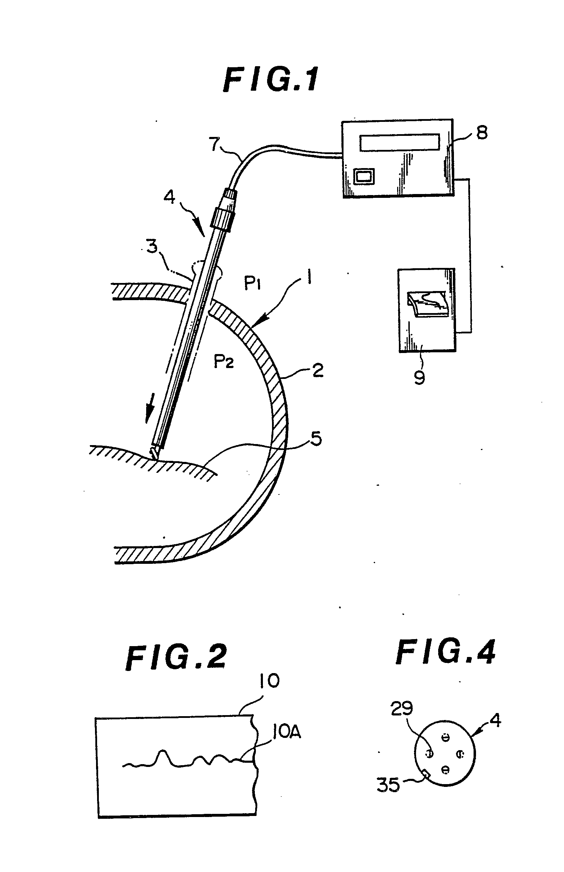

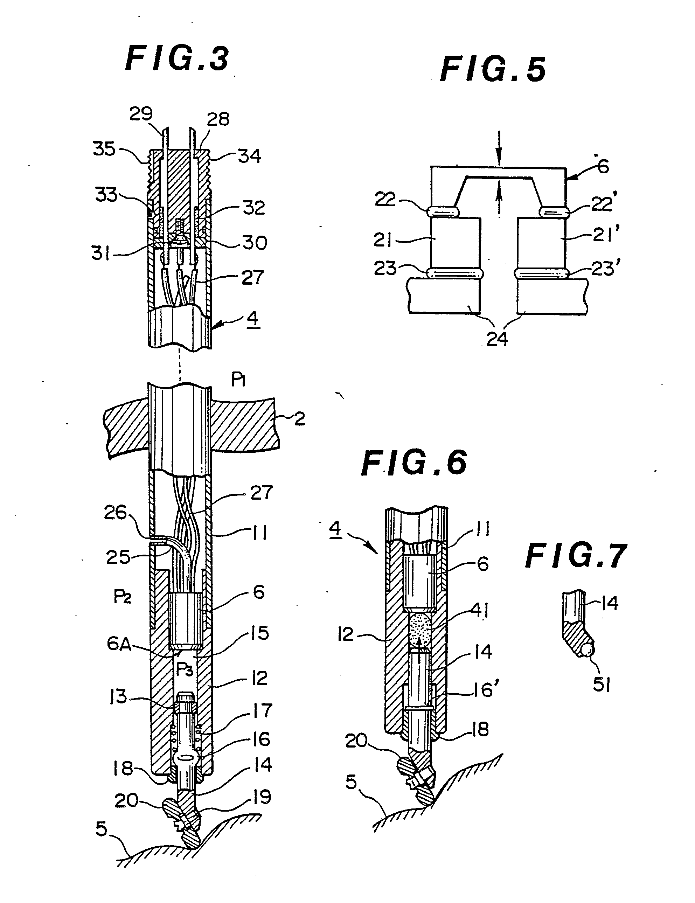

We will commence with a brief explanation of the hardness measuring operation. As shown in Figure 1, a hardness measuring probe 4 can be inserted through a trocar 3 in an abdominal wall 2 of a human body 1 until its tip is in contact with a living body structure 5, and if it is moved so as to rub the surface with a proper pressure, any delicate variations in hardness can be detected by a built-in pressure sensor 6 (Figure 3) and transmitted via a connecting head 7 to a measuring device 8 so that the peak value may be recorded and indicated in an indicating panel, or a hardness distribution curve 10A as shown in Figure 2 may be recorded on a sheet 10 by an XY plotter 9.

The first exemplary embodiment will be described in detail with reference to Figures 3 to 5. The hardness measuring probe 4 of the first exemplary embodiment as shown magnified in Figure 3, has a tubular, relatively thick tip portion 12 having a stepped internal diameter slightly expanded at each end and secured to an elongate outer tube 11 of the probe 4, and a pressure sensor 6 is fitted into the stepped bore and fixed by a bonding agent so that a fine diameter part of the bore, of fixed length, is provided in the tip portion 12 on the side facing a first pressure receiving surface 6A, and a piston shaft 14 fitted with a sealing member 13 is fitted in this fine diameter part. A pressure chamber 15 is thus formed in the hollow tip portion 12, sealed at each end by the above mentioned pressure sensor 6 and the inner end of the piston shaft 14, fitted with the sealing member 13, so that the pressure $P_3$ in this pressure chamber 15 may vary with the sliding movement (vertical movement in the illustration) of the piston shaft 14 and act on the exposed pressure receiving surface 6A of the pressure sensor 6.

The above mentioned piston shaft 14 has an annular projection 16 formed on the outer periphery substantially at its centre, and the tip portion 12 has an enlarged bore to provide a stepped recess of large diameter to contain this projection 16. A biasing coil spring 17 is fitted in the large diameter recess above the projection 16 and surrounding the piston shaft 14. This coil spring 17 is always in contact with the projection 16 of the piston shaft 14 so as to bias the piston shaft 14 to be pressed toward the tip (downward in the illustration), in the axial direction of the probe 4. To prevent this piston shaft 14 from springing out of the recess of the large diameter, a substantially ring-shaped retaining seal 18 having an internal diameter substantially equal to that of the diameter of the piston shaft 14 is fixed in the tip portion 12. A roller 20 is rotatably supported on a shaft pin 19 with one surface of the roller contacting a diagonally cut tip surface of the piston shaft 14, the pin 19 passing through a hole in the roller 20 into a bore in the above mentioned cut surface.

The basic structure of the pressure sensor 6 is shown in Figure 5. A semiconductor such as silicon is formed as a diaphragm type of pressure receiving surface for pressure detection by utilising the piezo-resistive effect so that the resistance of the semiconductor will vary with the forces acting on both the upper and lower surfaces and is secured at each end to respective base seats 21 and 21' by respective bonding agents 22 and 22', and these base seats are secured to a container 24 by respective bonding agents 23 and 23' to form a pressure sensor of the diaphragm type so that pressures acting on the above mentioned respective pressure receiving surfaces, as indicated by the arrows, may be detected as variations of the resistance values formed by the diaphragm type silicon, which is connected in a Wheatstone bridge.

In the pressure sensor 6, the pressure receiving surface 6A on one side faces the pressure chamber 15, and the pressure receiving surface on the other side receives a pressure $P_2$ from a communicating hole 26 provided in the outer periphery of the tube 11 via a fine guide tube 25 formed on the base of the pressure sensor 6, so that this pressure $P_2$ affects the measured result. The signals corresponding to the differences in pressure values acting on the pressure receiving surface 6A on one side and the pressure receiving surface on the other side are led to respective contacts 29 provided in a short columnar supporting member 28 fixed to the external end of the outer tube 11 through signal leads 27. In the supporting member 28, a lid 30 is fixed with a screw 31 or the like to the end surface on the side fitted in the outer tube and the respective bar-shaped contacts 29 projecting on both sides are pressed in and fixed through the supporting member 28 to which this lid 30 is fitted. The respective contacts 29 are cut to be step-shaped at both ends so that, at the lower end (in the illustration) when pressed in, the outer tube 11 to which the supporting member 28 is fitted will be positively sealed by respective sealing members 32.

The above mentioned supporting member 28 is fixed with a screw 33 or the like to the outer tube 11, a screw thread 34 is formed on the outer peripheral surface projecting upward from the outer tube 11, and an axial positioning groove 35 is formed at a point on the outer periphery of the upper end of the outer surface to orient the electrical contacts 29 and ensure that signals corresponding to the differing forces are transmitted to the measuring device 8 through the connecting leads 7 may be simultaneously indicated, the measured value on the other side being automatically deducted from the measured value on one side and the result indicated.

The operation of the thus formed first exemplary embodiment will now be further described.

First of all, the connecting leads 7 provided with respective connectors, as shown in Figure 1, is connected to the measuring device 8 for the hardness measuring probe 4, as described above, the hardness measuring probe 4 is inserted through the abdominal wall 2, guided by the trocar 3, and the roller 20 at the tip of the hardness measuring probe 4 is made to contact the surface of a living body structure 5 within a body cavity with appropriate pressure. The pressure sensor 6 used in this case is so made as to be able to measure the respective pressure values of both the pressure $P_3$ acting on one pressure receiving surface 6A, facing the pressure chamber 15, and also the pressure $P_2$ in the abdominal cavity (inflated with air) into which the probe is inserted which is led to the other pressure receiving surface through the fine tube 25. Therefore, the pressure difference values can be simultaneously indicated, or can be recorded by an XY plotter 9 or the like. It can be indicated on the plotter 9 that the pressure $P_2$ value on the other surface, read before the probe tip made contact with the surface of the structure 5, is deducted in advance from the reading obtained by pressure $P_3$ then measured on the pressure receiving surface 6A, and as shown in Figure 2, the subsequent measured results can be continuously recorded on a recording sheet by the XY plotter 9.

When the roller 20 is moved to rub on an object position and apply an appropriate force, the roller 20 will rotate and move, a force will act on the piston shaft 14 having the roller 20 pivoted at the tip, in response to the hardness of the position contacted by the roller 20, the biasing coil spring 17 will be extended or contracted to move the piston shaft 14 up or down vertically along the axial direction of the outer tube 11, this vertical movement will be transmitted to the pressure chamber 15 and the pressure $P_3$ of the pressure chamber 15 will be proportional to the force pressing the piston shaft 14 or the roller 20. Therefore, if the pressure $P_3$ of the pressure chamber 15, acting as shown by the arrow on the pressure receiving surface 6A on one side is measured, the hardness of the object position will be able to be continuously measured by a simple operation.

In this embodiment, as the pressure $P_2$ within the abdominal cavity can be measured, this pressure $P_2$ can be automatically deducted from the pressure difference reading obtained via the piston 14 and tube 25, and therefore the hardness of the object position can be easily measured at a high precision without being influenced by fluctuations of the pressure $P_2$ within the body cavity, which act both on the sensor and on the object surface 5, so that there is no need to measure by repeating contact and separation, as in the conventional example, and therefore the bad influence of any fluctuation of the pressing force between respective contact operations can be eliminated and the measuring precision can be improved. This helps to ensure that the delicate variations in hardness between an affected part and normal parts around the object position can be detected. Further, as the hardness measuring probe 4 is a sealed structure, it can be used in a wide range of applications, not only within an abdominal cavity inflated with air, but also within a bladder containing circulated water, and as the hardness measuring probe 4 can be separated from the connecting cord 7, it can be easily sterilised.

Because the roller 20 rotates in contact with the part contacting the object position, not only on a flat surface but also on an irregular surface, the hardness can be continuously measured with reduced variations of the pressing force. Also, as the sealed pressure chamber 15 is provided, the force acting on the pressure sensor 6 will be a uniform pressure and the pressure sensor 6 will be subjected to little wear, is not excessively deformed, and can be used for a long time. As the roller 20 contacting the object to be measured rotates whilst in contact, it is not subjected to wear or deformation and will have a relatively long working life.

Figure 6 shows a second exemplary embodiment wherein, instead of the biasing coil spring 17 used in the first exemplary embodiment, an elastic biasing member 41 having a predetermined softness is contained in the pressure chamber 15.

The elastic biasing member 41, moulded of a foam synthetic resin or rubber material, is in contact with the pressure receiving surface 6A on one side of the pressure sensor 6 and with the end face of the piston shaft 14, within the pressure chamber 15, so that the piston 14 is urged by this elastic biasing member 41 toward the tip in the axial direction of the outer tube 11. A flange-shaped projection 16' contacts the inner peripheral surface of the large diameter recess to regulate the movement of the piston shaft 14 that is guided by the retaining seal 18 that is fitted at the tip side as a stopper.

In this embodiment, when the elastic biasing member 41 is pressed and deformed, the relative hardness of the contacted object position will be transmitted to the pressure receiving surface 6A on one side of the pressure sensor 6 as indicated by the arrow. The operation and effectiveness of this second embodiment are substantially the same as described for the first embodiment.

Figure 7 shows a detail of a third exemplary embodiment, wherein a hemispherical or like recess is formed in the tip of the piston shaft 14 and a ball 51 is rotatably fitted therein instead of providing the roller 20.

The operation and effectiveness of this embodiment are the same as described for the two previously described embodiments, and this modification can be employed in either one of them.

In each of the above described embodiments, a single diaphragm type pressure sensor 6 is used, but in some cases, two ordinary plate-like pressure sensors can be used as the sensor means, a first pressure sensor being fixed to act in place of the diaphragm type pressure sensor surface 6A, and the pressure $P_2$ on the outer periphery of the outer tube 11 being applied to a pressure receiving surface of the second pressure sensor through a fine tube such as the tube 25 as is described above, or directly through an aperture such as the communicating hole 26 provided in the side of the outer tube 11. The ambient pressure $P_2$ will be measured and be indicated (recorded) in the same manner as in the value measured by the above mentioned first pressure sensor or the value measured by the second pressure sensor will be deducted from the value measured by the first pressure sensor and the influence caused by any pressure fluctuation will be automatically compensated to give a corrected indication.

The above described hardness measuring probes 4 can be used not only in the therapeutic field, but also in the industrial field.

Further, in each of the above described embodiments, the pressure from the measured object is made to act on the pressure receiving surface 6A via an elastic intermediate member, either a gas or resilient biasing solid. However, if only a slight pressing force is to be measured, the end face of the piston 14 can be made to act in direct contact with the pressure receiving surface 6A.

In each of the above described embodiments, a roller 20 or ball 51 rotatably mounted as a contact member is provided at the tip of the piston 14. However, in some cases the piston 14 may be moulded or machined to have a rounded tip portion that can directly slide in contact with the surface of the object to be measured.

The above described embodiments of the present invention can maintain a comparatively stable contact with the surface of the measured object, despite recesses and projections, and makes it possible to quickly and easily measure the hardness contours with a comparatively high precision.

In the embodiments of the present invention, the probe tip portion 12 must be formed of a hard member, but the outer tube 11 can be formed as a flexible member to facilitate insertion through a somewhat curved path to measure an internal hardness. Thus embodiments of the present invention can be adapted to a wide range of uses.

A hardness measuring probe constructed in accordance with the present invention can be inserted through the forceps channel of a hard endoscope or a soft endoscope, to measure the hardness of the inside wall of an internal organ within a body cavity.

Further, if the direction of fitting of the roller 20, the ball 51, or of the piston shaft 14 is varied or is made variable (part of the tip portion 12 may be made curvable from the base), the variations in hardness of the surface over a very wide region around the insertion sheath can be measured with high precision.

## Claims

1. A tubular probe (4) for sensing hardness variations when rubbed over a surface to be examined comprising pressure sensor means (6) to detect the difference in respective pressures applied at two pressure receiving surfaces, a slidable shaft (14) being provided at the distal end of the tubular probe to transmit the force applied to its tip by the rubbing to an inner end acting directly or indirectly on a first of said pressure receiving surfaces (6A), arranged to face the inner end of said shaft, and an aperture (26) on the peripheral surface of the probe communicating the ambient pressure around the probe with the second of said pressure receiving surfaces.

2. A probe as claimed in Claim 1, in which a rotatable member (20, 51) is mounted at the tip of said shaft.

3. A probe as claimed in Claim 2, in which said rotatable member is a roller (20).

4. A probe as claimed in Claim 2, in which said rotatable member is a ball (51).

5. A probe as claimed in any preceding Claim, in which said pressure sensor means (6) is a single sensor of a diaphragm type.

6. A probe as claimed in any preceding Claim, in which said shaft (14) is sealed on the outer periphery, within the probe.

7. A probe as claimed in any preceding Claim, in which a connector (28, 29) for a signal lead (27) transmitting the detected output signal is detachably connected to the end of the probe.

8. A probe as claimed in any preceding Claim, in which a sealed pressure chamber (15) is formed between the base end of said shaft and the first pressure receiving surface.

9. A probe as claimed in Claim 8, in which said pressure chamber (15) contains a gas.

10. A probe as claimed in Claim 8, in which said pressure chamber (15) contains a soft elastic biasing member (41).

## Patentansprüche

1. Rohrförmige Sonde (4) zum Bestimmen von Häreänderungen beim Reiben über eine zu prüfende Oberfläche, gekennzeichnet durch Drucksensormittel (6) zum Erfassen des Druckunterschiedes, der auf zwei druckaufnehmende Oberflächen aufgebracht wird, einem gleitenden Schaft (14), der am distalen Ende der rohrförmigen Sonde angeordnet ist und der die auf seine Spitze durch das Reiben aufgebrachte Kraft auf das innere Ende überträgt, das direkt oder indirekt auf eine erste der druckaufnehmenden Oberflächen (6A) einwirkt und die so angeordnet ist, daß die dem inneren Ende des Schaftes zugekehrt ist und durch eine Öffnung (26) an der peripheren Oberfläche der Sonde, die den Umgebungsdruck um die Sonde mit der zweiten druckaufnehmenden Oberfläche verbindet.

2. Sonde nach Anspruch 1, dadurch gekennzeichnet, daß an der Spitze des Schaftes ein drehbares Teil (20, 51) angeordnet ist.

3. Sonde nach Anspruch 3, dadurch gekennzeichnet, daß das drehbare Teil eine Rolle (20) ist.

4. Sonde nach Anspruch 2, dadurch gekennzeichnet, daß das drehbare Teil ein Ball (51) ist.

5. Sonde nach einem der vorstehenden Ansprüche 1—4, dadurch gekennzeichnet, daß die Drucksensormittel (6) aus einem einzelnen Sensor des Diaphragmatyps bestehen.

6. Sonde nach einem der vorstehenden Ansprüche 1—5, dadurch gekennzeichnet, daß der Schaft (14) an der äußeren Peripherie innerhalb der Sonde abgedichtet ist.

7. Sonde nach einem der vorstehenden Ansprüche 1—6, dadurch gekennzeichnet, daß ein Verbinder (28, 29) für ein Signalkabel (27), das das aufgenommene Ausgangssignal überträgt, lösbar mit dem Ende der Sonde verbunden ist.

8. Sonde nach einem der vorstehenden Ansprüche 1—7, dadurch gekennzeichnet, daß eine abgedichtete Druckkammer (15) zwischen dem Basisende des Schaftes und der ersten druckaufnehmenden Oberfläche vorgesehen ist.

9. Sonde nach Anspruch 8, dadurch gekennzeichnet, daß die Druckkammer (15) ein Gas enthält.

10. Sonde nach Anspruch 8, dadurch gekennzeichnet, daß die Druckkammer (15) ein weiches, elastisches vorgespanntes Teil enthält.

## Revendications

1. Sonde tubulaire (4) pour détecter les variations de dureté sur une surface à examiner comprenant des moyens détecteurs de pression (6) pour détecter la différence des pressions respectives appliquées sur deux surfaces soumises à une pression, un arbre coulissant (14) étant prévu à l'extrémité distale de la sonde tubulaire pour transmettre la force appliquée par le frottement sur sa pointe à une extrémité intérieure agissant directement ou indirectement sur une première desdites surfaces (6A) soumises à une pression, disposée de façon à être tournée vers l'extrémité interne dudit arbre, et une ouverture (26) sur la surface périphérique de la sonde communiquant la pression ambiante autour de la sonde à la seconde desdites surfaces soumises à une pression.

2. Sonde selon la revendication 2, caractérisée en ce qu'un élément tournant (20, 51) est monté à l'extrémité dudit arbre.

3. Sonde selon la revendication 2, caractérisée en ce que ledit élément tournant est un galet (20).

4. Sonde selon la revendication 2, caractérisée en ce que ledit élément tournant est une bille (51).

5. Sonde selon l'une quelconque des revendications précédentes, caractérisée en ce que lesdits moyens capteurs de pression sont constitués d'un unique capteur du type à diaphragme.

6. Sonde selon l'une quelconque des revendications précédentes, caractérisée en ce que ledit arbre (14) est scellé sur sa périphérie extérieure, à l'intérieur de la sonde.

7. Sonde selon l'une quelconque des revendications précédentes, caractérisée en ce qu'un connecteur (28, 29) pour une connexion de signal (27) transmettant le signal de sortie détecté est connecté de façon amovible à l'extrémité de la sonde.

8. Sonde selon l'une quelconque des revendications précédentes, caractérisé en ce qu'une chambre sous pression, fermée (15) est formée entre l'extrémité de base dudit arbre et ladite première surface soumise à une pression.

9. Sonde selon la revendication 8, caractérisée en ce que ladite chambre sous pression (15) contient un gaz.

10. Sonde selon la revendication 8, caractérisée en ce que ladite chambre sous pression (15) contient une pièce élastique souple servant de poussoir.

# FIG.1

# FIG.2

# FIG.4

# FIG.3

# FIG.5

# FIG.6

# FIG.7